Europäisches Patentamt

European Patent Office

Office européen des brevets

(19)

(11) **EP 1 700 576 A1**

(12) **EUROPÄISCHE PATENTANMELDUNG**

(43) Veröffentlichungstag:
**13.09.2006 Patentblatt 2006/37**

(51) Int Cl.:
*A61C 13/00* (2006.01)  *A61C 19/05* (2006.01)

(21) Anmeldenummer: **06110811.4**

(22) Anmeldetag: **08.03.2006**

(84) Benannte Vertragsstaaten:
**AT BE BG CH CY CZ DE DK EE ES FI FR GB GR
HU IE IS IT LI LT LU LV MC NL PL PT RO SE SI
SK TR**
Benannte Erstreckungsstaaten:
**AL BA HR MK YU**

(30) Priorität: **08.03.2005 DE 102005011066**

(71) Anmelder: **Sirona Dental Systems GmbH
64625 Bensheim (DE)**

(72) Erfinder:
• **Orth, Ulrich
  64686, Lautertal (DE)**
• **Wedler, Volker
  69493, Hirschberg (DE)**

(74) Vertreter: **Sommer, Peter
Patentanwalt
Viktoriastrasse 28
68165 Mannheim (DE)**

(54) **Verfahren zur Herstellung der Lageübereinstimmung von 3D-Datensätzen in einem dentalen CAD/CAM-System**

(57) Es wird ein Verfahren zur Konstruktion von Zahnoberflächen eines als 3D-Datensatzes vorliegenden digitalen Zahnersatzteils unter Verwendung eines 3D-Modells eines Präparationsgebiets und/oder eines Zahnersatzteils und eines weiteren 3D-Modells, welches mit dem ersten 3D-Modell übereinstimmende Bereiche aufweist, wobei auf dem ersten 3D-Modell A und dem zweiten 3D-Modell A' aus dem übereinstimmenden Bereich mindestens drei Paare ($P_1$, $P_2$, $P_3$) voneinander entsprechenden Punkten ($P_{11}$, $P_{12}$; $P_{21}$, $P_{22}$; $P_{31}$, $P_{32}$) festgelegt werden und anhand der mindestens drei Paare ($P_1$, $P_2$, $P_3$) die Lagebeziehung des zweiten 3D-Modells bezüglich des ersten 3D-Modells bestimmt wird.

**Fig. 1**

**Beschreibung**

Technisches Gebiet

Die Erfindung betrifft einen Teil eines Verfahrens zur Herstellung von Zahnersatzteilen in einem dentalen CAD-System.

Stand der Technik

Im dentalen CAD/CAM-System "CEREC", eingetragene Marke der Sirona Dental Systems GmbH, Deutschland, führt der Benutzer eine 3D-Vermessung eines präparierten Zahnes und seiner Nachbarzähne durch und daraus wird im Speicher eines Rechners eine Datenrepräsentation des 3D-Modells des aufgenommenen Gebietes erstellt und angezeigt.

Um z.B. die Kaufläche eines Zahns vor der Präparation zu kopieren, kann der Zahn vor der Präparation gemeinsam mit den Nachbarzähnen aufgenommen werden und ein entsprechendes 3D-Modell berechnet werden.

Eine weitere Variante ist das Aufnehmen eines Abdrucks des Gegenkiefers, um das Zahnersatzteil optimal an den Gegenkiefer anpassen zu können.

Um die Information des nicht präparierten Zahns bzw. des Abdrucks des Gegenkiefers nutzen zu können, muss eine hinreichend exakte geometrische Zuordnung der entsprechenden Modelle zum Modell des präparierten Zahns erfolgen. In den Modellen müssen daher Informationen enthalten sein, die diese Zuordnung ermöglichen.

Es ist bekannt, die Oberfläche der Nachbarzähne zu verwenden, die sich zwischen den Aufnahmen nicht verändert hat. Im Normalfall erfolgt dies automatisch durch die Software, indem einander entsprechende Gebiete des 3d-Datensatzes gesucht und gefunden werden.

Es gibt Fälle, in denen, bedingt z.B. durch die Qualität der Aufnahmen, zu geringe nicht veränderte Anteile in den 3D-Modellen oder störende Bildbestandteile wie Kofferdamm oder Zellstoffrollen, dieser automatische Prozess scheitert.

Um trotzdem die Information aus den Aufnahmen vor der Präparation oder aus dem Gegenkiefer verwenden zu können, muss die Lageübereinstimmung der Modelle auf andere Art hergestellt werden.

Darstellung der Erfindung

Die Erfindung betrifft ein Verfahren zur Konstruktion von Zahnoberflächen eines als 3D-Datensatz vorliegenden digitalen Zahnersatzteils unter Verwendung eines 3D-Modells eines Präparationsgebiets und/oder eines noch zu bearbeitenden Zahnersatzteils und eines weiteren 3D-Modells, welches mit dem ersten 3D-Modell übereinstimmende Bereiche aufweist. Auf dem ersten 3D-Modell A und dem zweiten 3D-Modell A' werden aus dem übereinstimmenden Bereich mindestens drei Paare von einander entsprechenden Punkten festgelegt und anhand der mindestens drei Paare wird die Lagebeziehung des zweiten 3D-Modells bezüglich des ersten 3D-Modells bestimmt.

Mittels der Lagebeziehung ist eine Zuordnung von Punkten und Bereichen des zweiten 3D-Modells und des ersten 3D-Modells bekannt.

Die Zuordnung der Punkte erfolgt durch einen Eingriff des Benutzers.

Die Erfindung betrifft weiterhin ein Verfahren zur Konstruktion von Zahnoberflächen eines als 3D-Datensatz vorliegenden digitalen Zahnersatzteils, unter Verwendung eines 3D-Modells eines Präparationsgebiets und/oder eines noch zu bearbeitenden Zahnersatzteils und eines weiteren 3D-Modells, welches mit dem ersten 3D-Modell übereinstimmende Bereiche aufweist. Auf dem ersten 3D-Modell und dem zweiten 3D-Modell werden aus dem übereinstimmenden Bereich dass mehrere Punkte ausgewählt die zueinander benachbart sind und einen Bereich der Oberfläche bilden und anhand der mehreren Punkte in dem Bereich wird die Lagebeziehung des zweiten 3D-Modells bezüglich des ersten 3D-Modells bestimmt.

Vorteilhafterweise werden dabei mehrere Bereiche mit mehreren Punkten bestimmt werden.

Eine Weiterbildung der Erfindung besteht darin, für die Bestimmung der Lagebeziehung die Summe der Abstände der festgelegten Punkte zu minimieren.

Die Erfindung umfasst weiterhin ein Verfahren zur Konstruktion von Zahnoberflächen eines als 3D-Datensatz vorliegenden digitalen Zahnersatzteils, unter Verwendung eines 3D-Modells eines Präparationsgebiets und/oder eines noch zu bearbeitenden Zahnersatzteils und eines weiteren 3D-Modells, welches mit dem ersten 3D-Modell übereinstimmende Bereiche aufweist. Auf dem ersten 3D-Modell und dem zweiten 3D-Modell wird aus dem übereinstimmenden Bereich ein Paar von einander entsprechenden Punkten festgelegt und das zweite 3D-Modell mit dem ersten 3D-Modell an diesem Punkt zur Überdeckung gebracht und angezeigt wird. Das 3D-Modell ist dann gegenüber dem Zahnersatzteil über Eingabemittel um den mindestens einen Punkt verstellbar und anhand einer eingestellten Lage wird eine Lagebeziehung ermittelt. Sowohl die Auswahl des Punktes als auch die Verstellung erfolgt durch den Benutzer.

Gemäß der Erfindung wird anhand der festgestellten Lagebeziehung eine erste Transformation durchgeführt.

Eine besonders vorteilhafte Weiterbildung besteht darin, diejenigen Bereiche, die nur eine geringe Höhendifferenz zwischen dem ersten 3D-Modell und dem zweiten 3D-Modell aufweisen, als Grundlage für die Ermittlung einer weiteren Lagebeziehung zu verwenden.

Vorteilhafterweise wird anhand der weiteren Lagebeziehung eine zweite Transformation durchgeführt wird.

Kurzbeschreibung der Zeichnung

In der Zeichnung sind Ausführungsbeispiele der Erfindung dargestellt. Es zeigt die

Fig. 1      zwei 3D-Modelle A und A', die
Fig. 2      die Lagebeziehung in abstrakter Weise durch ein erstes Koordinatensystem K1 und ein zweites Koordinatensystem K2, die
Fig. 3a     ein anderes Verfahren zur Herstellung einer Lagebeziehung mit einem ersten Schritt, die
Fig. 3b     eine Verdrehung des Koordinatensystems K2' aus Fig. 3a, die
Fig. 4      eine erste Optimierung der Bestimmung der Lagebeziehung und die
Fig. 5      eine weitere Optimierung der Bestimmung der Lagebeziehung.

Ausführungsbeispiel der Erfindung

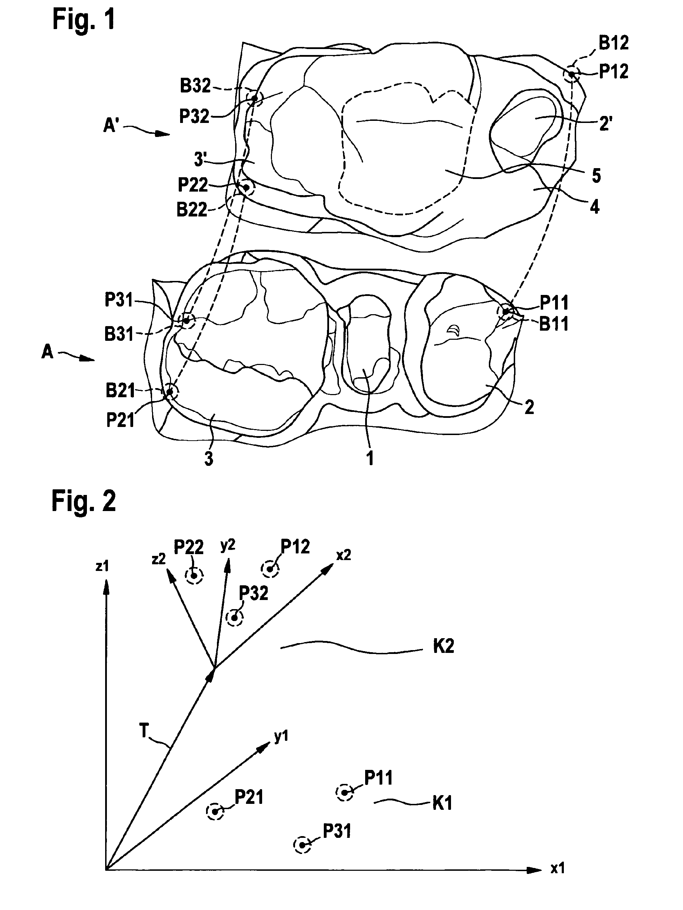

In Fig. 1 sind zwei 3D-Modelle A und A' gezeigt. Das 3D-Modell A ist eine Datenrepräsentation eines präparierten Zahns 1 mit den Nachbarzähnen 2, 3, das 3D-Modell A' ist eine Datenrepräsentation in etwa desselben Präparationsgebiets, wobei allerdings in einer Abdruckmasse 4 ein Abdruck 5 des Gegenkiefers enthalten ist und wobei die Nachbarzähne 2', 3' durch die Abdruckmasse 4 stark überdeckt werden. Teile des Abdrucks 5 sollen bei der Gestaltung eines Zahnersatzteils berücksichtigt werden, hier die Kaufläche im Abdruck 5. Beide 3D-Modelle A,A' sind bislang noch ohne räumlichen Bezug zueinander. Man erkennt mit bloßem Auge durch Vergleich markanter Oberflächenpunkte oder Bereiche, dass in beiden Modellen einander entsprechende Oberflächenpunkte oder Bereiche der Oberfläche vorhanden sind. Diese Oberflächen sind insbesondere Kanten oder Höcker.

Beide 3D-Modelle A, A' sind in der gleichen Vergrößerung dargestellt und stimmen bezüglich ihrer dargestellten Abmessungen folglich überein. Auch eine Darstellung der Modelle in unterschiedlichem Maßstab ist grundsätzlich möglich, da die dargestellten Bereiche immer auf absoluten Daten beruhen.

Der Benutzer kann daher Punkte $P_{11}$-$P_{32}$ oder Bereiche $B_{11}$-$B_{32}$ von benachbarten Punkten definieren, die in beiden 3D-Modellen A, A' die gleichen Objektteile, in diesem Fall Teile der Nachbarzähne 2, 2'; 3, 3', abbilden.

Durch Markierung von Punktepaaren $P_{11}$, $P_{12}$; $P_{21}$, $P_{22}$; $P_{31}$, $P_{32}$, wobei jeweils ein Punkt $P_{11}$, $P_{21}$, $P_{31}$ eines Paares auf dem 3D-Modell A und der andere Punkt $P_{12}$, $P_{22}$, $P_{32}$ des Paares auf dem 3D-Modell A' liegt, lässt sich mit bekannten mathematischen Verfahren eine eindeutige Lagebeziehung herstellen. Dies ist selbst dann möglich, wenn unterschiedlich große Darstellungen vorliegen.

In Fig. 2 ist diese Lagebeziehung in abstrakter Weise durch ein erstes Koordinatensystem K1 und ein zweites Koordinatensystem K2 und eine Transformation T zur Angabe der Lagebeziehung dargestellt. In beidem Koordinatensystemen sind Punktepaare $(P_{11}, P_{12})$, $(P_{21}, P_{22})$ $(P_{31}, P_{32})$ festgelegt, aus denen die Transformation T berechnet wird. Damit lässt sich das Koordinatensystem K2 gemäß einer mathematischen Vorschrift wie folgt auf das Koordinatensystem K1 abbilden:

$$K_1 = T * K_2$$

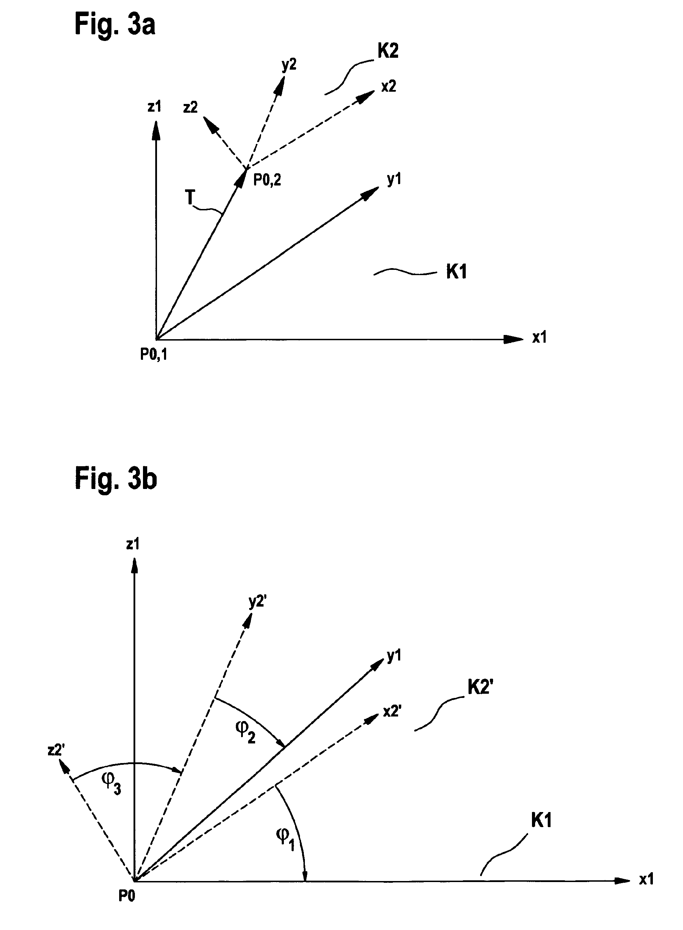

In Fig. 3a ist ein anderes Verfahren zur Herstellung einer Lagebeziehung zwischen zwei Koordinatensystemen K2, K2 dargestellt. Zunächst wird anhand eines Punktepaares $(P_{01}, P_{02})$ eine erste Transformation T berechnet, die den Punkt $P_{02}$ auf den Punkt $P_{01}$ abbildet und ausschließlich einen Translationsanteil enthält.

Anschließend erfolgt durch den Benutzer eine Verdrehung des Koordinatensystems K2' um den gemeinsamen Punkt $P_0$ um die Winkel $\varphi_1$, $\varphi_2$, $\varphi_3$ der Achsen x, y und z, dargestellt in Fig. 3b, um eine Überdeckung der beiden Koordinatensysteme K1, K2 im Koordinatensystem K1 herbeizuführen. Daraus wird eine Transformation R bestimmt, die nur die Drehwinkel und keine Translationsanteile enthält. Schließlich wird eine Lagebeziehung für die Koordinatensysteme K1, K2 berechnet.

$$K_1 = R * (T * K_2)$$

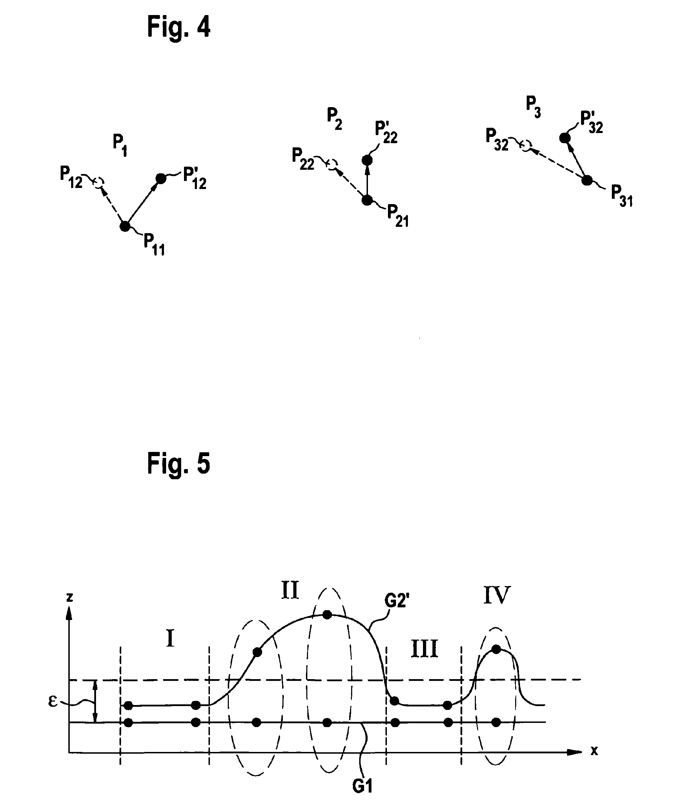

In Fig. 4 ist dargestellt, wie die Bestimmung der Lagebeziehung der drei Punktepaare $P_1, P_2, P_3$ erfolgt. Dazu wird die Transformation gesucht, bei der die drei Punktepaare $P_1$, $P_2$, $P_3$ so zueinander angeordnet sind, dass der Abstand der Punkte $(P_{11}, P_{12}')$, $(P_{21}, P_{22}')$ und $(P_{31}, P_{32}')$ nach der Transformation T der Punkte $P_{12}$, $P_{22}$ und $P_{32}$ des 3D-Modells A' auf die Punkte $P_{12}'$, $P_{22}'$ und $P_{32}'$ minimal ist. Dies geschieht mit an sich bekannten Algorithmen unter Anwendung der folgenden Formel, wobei im vorliegenden Fall n=3 ist:

$$\min \sum_{i=1}^{n} \left\| P_{i1} - T \cdot P_{i2} \right\|$$

Als Folge dieser so ermittelten Transformation T werden also die Punkte $P_{12}$, $P_{22}$ und $P_{32}$ auf die Punkte $P_{12}'$, $P_{22}'$ und $P_{32}'$ abgebildet und diese Transformation kann auch auf das übrige 3D-Modell A' angewendet werden, um die Lagebeziehung einzelner Punkte oder ausgewählter Bereiche herzustellen.

Für einen aus mehreren Punkten bestehenden Bereich gilt entsprechendes.

Eine Optimierung ist in Fig. 5 dargestellt. Ausgehend von der Überlegung, dass nach Durchführung der ersten Transformation Bildbereiche mit geringen Änderungen zwischen den beiden Modellen dicht beieinander liegen, also einen geringen Abstand aufweisen, werden für die weitere Optimierung der Transformation diejenigen Bereiche ausgeblendet, die einen vorgegebenen Höchstabstand überschreiten.

Zusammen mit einer Kurve G1 in der x,z-Ebene aus dem ersten Koordinatensystem als Beispiel für den Verlauf einer Oberfläche eines Zahnersatzteils als 3D-Modell ist eine Kurve G2' dargestellt, die aus einer nicht dargestellten Kurve G2 aus dem zweiten Koordinatensystem nach der ersten Transformation entstanden ist. Der Verlauf der Kurve G2' weist Bereiche I, III auf, die innerhalb eines Höchstwerts ε zu der Kurve G1 beabstandet sind, wohingegen in den Bereichen II und IV ist die Abweichung jedoch deutlich größer als der Höchstwert ε ist. Diese Bereiche II und IV werden als Störungen angesehen und bleiben bei der Berechnung der endgültigen Transformation außer Betracht. Die Lagebeziehung wird daher nur mit den innerhalb der Höchstgrenze ε liegenden Werten der Bereiche I und III berechnet.

Idealerweise liegen die Kurven im Bereich I und III nach dieser weiteren Transformation aufeinander.

Es ist ohne weiteres verständlich, dass die Punktepaare für die erste Transformation in den Bereichen I und III zu liegen kommen sollten, damit diese gute Übereinstimmung erreicht wird.

**Patentansprüche**

1. Verfahren zur Konstruktion von Zahnoberflächen eines als 3D-Datensatz vorliegenden digitalen Zahnersatzteils unter Verwendung eines 3D-Modells eines Präparationsgebiets und/oder eines Zahnersatzteils und eines weiteren 3D-Modells, welches mit dem ersten 3D-Modell übereinstimmende Bereiche aufweist, **dadurch gekennzeichnet, dass** auf dem ersten 3D-Modell (A) und dem zweiten 3D-Modell (A') aus dem übereinstimmenden Bereich mindestens drei Paare $(P_1, P_2, P_3)$ von einander entsprechenden Punkten $(P_{11}, P_{12}; P_{21}, P_{22}; P_{31}, P_{32})$ festgelegt werden und dass anhand der mindestens drei Paare $(P_1, P_2, P_3)$ die Lagebeziehung des zweiten 3D-Modells bezüglich des ersten 3D-Modells bestimmt wird.

2. Verfahren zur Konstruktion von Zahnoberflächen eines als 3D-Datensatz vorliegenden digitalen Zahnersatzteils, unter Verwendung eines 3D-Modells eines Präparationsgebiets und/oder eines Zahnersatzteils und eines weiteren 3D-Modells, welches mit dem ersten 3D-Modell übereinstimmende Bereiche aufweist, **dadurch gekennzeichnet, dass** auf dem ersten 3D-Modell und dem zweiten 3D-Modell aus dem übereinstimmenden Bereich dass mehrere Punkte ausgewählt werden, die zueinander benachbart sind und einen Bereich $(B_{11}, B_{12}; B_{21}, B_{22}; B_{31}, B_{32})$ der Oberfläche bilden und dass anhand der mehreren Punkte in dem Bereich $(B_{11}, B_{12}; B_{21}, B_{22}; B_{31}, B_{32})$ die Lagebeziehung des zweiten 3D-Modells bezüglich des ersten 3D-Modells bestimmt wird.

3. Verfahren nach Anspruch 2, **dadurch gekennzeichnet, dass** mehrere Bereiche $(B_{11}-B_{32})$ mit mehreren Punkten bestimmt werden.

4. Verfahren nach einem der Ansprüche 1 bis 3, **dadurch gekennzeichnet, dass** für die Bestimmung der Lagebeziehung die Summe der Abstände der Punkte minimiert wird.

5. Verfahren zur Konstruktion von Zahnoberflächen eines als 3D-Datensatz vorliegenden digitalen Zahnersatzteils, unter Verwendung eines 3D-Modells eines Präparationsgebiets und/oder eines Zahnersatzteils und eines weiteren 3D-Modells, welches mit dem ersten 3D-Modell übereinstimmende Bereiche aufweist, **dadurch gekennzeichnet, dass** auf dem ersten 3D-Modell und dem zweiten 3D-Modell aus dem übereinstimmenden Bereich ein Paar von einander entsprechenden Punkten ($P_{01}$, $P_{02}$) festgelegt werden dass das zweite 3D-Modell mit dem ersten 3D-Modell an diesem Punkt zur Überdeckung gebracht und angezeigt wird und dass das 3D-Modell gegenüber dem Zahnersatzteil über Eingabemittel um den mindestens einen Punkt verstellbar ist und dass anhand einer eingestellten Lage eine Lagebeziehung ermittelt wird.

6. Verfahren nach einem der Ansprüche 1 bis 5, **dadurch gekennzeichnet, dass** anhand der festgestellten Lagebeziehung eine erste Transformation durchgeführt wird.

7. Verfahren nach Anspruch 6, **dadurch gekennzeichnet, dass** diejenigen Bereiche (I,III), die nach der ersten Transformation nur eine geringe Höhendifferenz ($\varepsilon$) zwischen dem ersten 3D-Modell (A) und dem zweiten 3D-Modell (A,) aufweisen, als Grundlage für die Ermittlung einer weiteren Lagebeziehung verwendet werden.

8. Verfahren nach Anspruch 7, **dadurch gekennzeichnet, dass** anhand der weiteren Lagebeziehung eine zweite Transformation durchgeführt wird.

# Fig. 1

# Fig. 2

# Fig. 3a

# Fig. 3b

# Fig. 4

$P_1$

$P_{12}$    $P'_{12}$

$P_{11}$

$P_2$

$P'_{22}$

$P_{22}$

$P_{21}$

$P_3$

$P'_{32}$

$P_{32}$

$P_{31}$

# Fig. 5

z

II    G2'

IV

I

III

ε

x

G1

Europäisches
Patentamt

**EUROPÄISCHER RECHERCHENBERICHT**

Nummer der Anmeldung

EP 06 11 0811

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 6 152 731 A (JORDAN ET AL) 28. November 2000 (2000-11-28) * Spalte 1, Zeilen 15-21,35-44 * * Spalte 5, Zeilen 34-51; Abbildungen 7,12A-B,14,16A-B * * Spalte 6, Zeilen 9-17 * * Spalte 7, Zeile 65 - Spalte 10, Zeile 15 * * Spalte 12, Zeilen 1-10 * * Spalte 17, Zeilen 40-65 * * Spalte 20, Zeile 12 - Spalte 21, Zeile 11 * * Spalte 22, Zeile 18 - Spalte 24, Zeile 54 * ----- | 1-6,8 | INV. A61C13/00 A61C19/05 |
| X | US 6 334 853 B1 (KOPELMAN AVI ET AL) 1. Januar 2002 (2002-01-01) * Spalte 2, Zeile 35 - Spalte 3, Zeile 5; Abbildungen 1,2,8 * * Spalte 6, Zeilen 9-17 * * Spalte 6, Zeile 54 - Spalte 7, Zeile 12; Anspruch 12 * * Spalte 7, Zeilen 33-40 * ----- | 1-8 | RECHERCHIERTE SACHGEBIETE (IPC) A61C |
| X | US 2002/094509 A1 (DURBIN DUANE ET AL) 18. Juli 2002 (2002-07-18) * Absätze [0009], [0010], [0022] - [0030], [0043], [0044], [0059], [0067] - [0070]; Abbildungen 1-4,9 * ----- | 1,5,6 | |
| X | US 5 027 281 A (REKOW ET AL) 25. Juni 1991 (1991-06-25) * Spalte 3, Zeilen 43-66 * * Spalte 8, Zeile 6 - Spalte 10, Zeile 37; Abbildungen 7-14 * * Spalte 12, Zeilen 33-51 * * Spalte 13, Zeile 53 - Spalte 15, Zeile 16; Abbildungen 23-25 * ----- -/-- | 1,2,6 | |

Der vorliegende Recherchenbericht wurde für alle Patentansprüche erstellt

| Recherchenort | Abschlußdatum der Recherche | Prüfer |
|---|---|---|
| München | 13. Juni 2006 | Pypen, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

 

& : Mitglied der gleichen Patentfamilie, übereinstimmendes Dokument

EPO FORM 1503 03.82 (P04C03)

**Europäisches Patentamt**

## EUROPÄISCHER RECHERCHENBERICHT

Nummer der Anmeldung

EP 06 11 0811

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2004/172150 A1 (PEROT JEAN-MARC ET AL) 2. September 2004 (2004-09-02) * Absätze [0002], [0010], [0015] - [0018], [0042], [0043], [0050] - [0052], [0057]; Ansprüche 13-15; Abbildungen 7,12,13 * ----- | 1-4,6,7 | |
| X | DE 196 42 247 C1 (MELLER, SEBASTIAN, 91054 ERLANGEN, DE) 15. Januar 1998 (1998-01-15) * Spalte 3, Zeile 63 - Spalte 4, Zeile 49; Abbildungen 1,2,5,8 * * Spalte 5, Zeilen 8-19 * * Spalte 7, Zeile 41 - Spalte 9, Zeile 1 * * Spalte 9, Zeilen 47-62 * ----- | 1-3 | |
| X | US 4 183 139 A (TANAKA, ASAMI) 15. Januar 1980 (1980-01-15) * Spalte 2, Zeile 55 - Spalte 4, Zeile 12; Abbildungen 4-7 * ----- | 1,2,5 | |
| A | WO 2005/004742 A (DEGUDENT GMBH; VON SCHROETER, PHILIP; ZOELLNER, MICHAEL) 20. Januar 2005 (2005-01-20) * das ganze Dokument * ----- | 6-8 | RECHERCHIERTE SACHGEBIETE (IPC) |
| E | US 2006/063135 A1 (MEHL ALBERT) 23. März 2006 (2006-03-23) * das ganze Dokument * ----- | 1 | |

Der vorliegende Recherchenbericht wurde für alle Patentansprüche erstellt

| Recherchenort | Abschlußdatum der Recherche | Prüfer |
|---|---|---|
| München | 13. Juni 2006 | Pypen, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................................

& : Mitglied der gleichen Patentfamilie, übereinstimmendes Dokument

EPO FORM 1503 03.82 (P04C03)

EP 1 700 576 A1

## ANHANG ZUM EUROPÄISCHEN RECHERCHENBERICHT
## ÜBER DIE EUROPÄISCHE PATENTANMELDUNG NR.

EP 06 11 0811

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-06-2006

| Im Recherchenbericht angeführtes Patentdokument | Datum der Veröffentlichung | Mitglied(er) der Patentfamilie | Datum der Veröffentlichung |
|---|---|---|---|
| US 6152731 A | 28-11-2000 | AU 6241898 A<br>DE 1017332 T1<br>EP 1017332 A1<br>JP 2001517480 T<br>JP 2005193028 A<br>WO 9915100 A1<br>US 6322359 B1 | 12-04-1999<br>19-07-2001<br>12-07-2000<br>09-10-2001<br>21-07-2005<br>01-04-1999<br>27-11-2001 |
| US 6334853 B1 | 01-01-2002 | AT 247433 T<br>AU 7231698 A<br>DE 69817342 D1<br>DE 69817342 T2<br>EP 0984739 A1<br>WO 9852493 A1<br>IL 120892 A<br>JP 2001525715 T | 15-09-2003<br>11-12-1998<br>25-09-2003<br>19-02-2004<br>15-03-2000<br>26-11-1998<br>31-08-2000<br>11-12-2001 |
| US 2002094509 A1 | 18-07-2002 | KEINE | |
| US 5027281 A | 25-06-1991 | KEINE | |
| US 2004172150 A1 | 02-09-2004 | WO 02102270 A1<br>CA 2350849 A1<br>DE 20220873 U1 | 27-12-2002<br>15-12-2002<br>22-04-2004 |
| DE 19642247 C1 | 15-01-1998 | KEINE | |
| US 4183139 A | 15-01-1980 | KEINE | |
| WO 2005004742 A | 20-01-2005 | AU 2004255445 A1<br>EP 1643926 A1 | 20-01-2005<br>12-04-2006 |
| US 2006063135 A1 | 23-03-2006 | AU 2003294708 A1<br>CA 2505892 A1<br>DE 10252298 B3<br>WO 2004044787 A2<br>EP 1581896 A2 | 03-06-2004<br>27-05-2004<br>19-08-2004<br>27-05-2004<br>05-10-2005 |

EPO FORM P0461

Für nähere Einzelheiten zu diesem Anhang : siehe Amtsblatt des Europäischen Patentamts, Nr.12/82